# EUROPEAN PATENT APPLICATION

(11) **EP 2 762 153 A2**
(43) Date of publication of application: **06.08.2014**
(21) Application number: 14000318.7
(22) Date of filing: 29.01.2014
(51) Int. Cl.: A61K 39/00

(54) **Vaccine composition for mucosal administration**

(30) Priority: 05.02.2013 JP 2013020910
(71) Applicant: NITTO DENKO CORPORATION, Osaka 567 (JP)
(72) Inventor: Okazaki, Arimichi, Ibaraki-shi Osaka 567-8680 (JP); Matsushita, Kyohei, Ibaraki-shi Osaka 567-8680 (JP); Asari, Daisuke, Ibaraki-shi Osaka 567-8680 (JP); Shishido, Takuya, Ibaraki-shi Osaka 567-8680 (JP); Maeda, Yoshiki, Ibaraki-shi Osaka 567-8680 (JP); Okubo, Katsuyuki, Ibaraki-shi Osaka 567-8680 (JP); Li, Wenjing, Ibaraki-shi Osaka 567-8680 (JP); Hori, Mitsuhiko, Ibaraki-shi Osaka 567-8680 (JP); Sugiyama, Haruo, Suita-shi Osaka 565-0871 (JP)
(74) Representative: Müller-Boré & Partner Patentanwälte PartG mbB

(57) **Abstract**

The present invention provides a vaccine composition which comprises a cellular immunity induction promoter universally usable against various antigens in cellular immunity induction by mucosal administration of the antigen and exerts a high cellular immunity inducing effect by mucosal administration. The present invention provides a vaccine composition for mucosal administration to induce cellular immunity, comprising: (i) an antigen; and (ii) one or more cellular immunity induction promoters selected from the group consisting of a TLR ligand, a cyclic dinucleotide, a helper peptide and an immunomodulatory small molecule drug.

SEQUENCE LISTING

<110>  NITTO DENKO CORPORATION

<120>  VACCINE COMPOSITION FOR MUCOSAL ADMINISTRATION

<130>  NITTO 7

<150>  JP 2013-020910
<151>  2013-02-05

<160>  15

<170>  PatentIn version 3.2

<210>  1
<211>  9
<212>  PRT
<213>  Homo sapiens

<400>  1

Ala Tyr Ala Cys Asn Thr Ser Thr Leu
1               5

<210>  2
<211>  9
<212>  PRT
<213>  Homo sapiens

<400>  2

Glu Tyr Ile Leu Ser Leu Glu Glu Leu
1               5

<210>  3
<211>  9
<212>  PRT
<213>  Homo sapiens

<400>  3

Thr Tyr Leu Pro Thr Asn Ala Ser Leu
1               5

<210>  4
<211>  9
<212>  PRT
<213>  Homo sapiens

<400>  4

Ile Met Pro Lys Ala Gly Leu Leu Ile
1               5

<210>  5
<211>  9
<212>  PRT
<213>  hepatitis C virus

<400>  5

Asp Leu Met Gly Tyr Ile Pro Ala Val
1               5

<210>  6
<211>  9
<212>  PRT
<213>  Homo sapiens

<400>  6

Val Leu Gln Glu Leu Asn Val Thr Val
1               5

<210>  7
<211>  9
<212>  PRT
<213>  Homo sapiens

<400>  7

Lys Val Phe Gly Ser Leu Ala Phe Val
1               5

<210>  8
<211>  9
<212>  PRT
<213>  Homo sapiens

<400>  8

Lys Val Ala Glu Ile Val His Phe Leu
1               5

<210>  9

<211>  9
<212>  PRT
<213>  hepatitis B virus

<400>  9

Trp Leu Ser Leu Leu Val Pro Phe Val
1               5


<210>  10
<211>  9
<212>  PRT
<213>  Homo sapiens

<400>  10

Lys Ile Phe Gly Ser Leu Ala Phe Leu
1               5


<210>  11
<211>  9
<212>  PRT
<213>  Homo sapiens

<400>  11

Ser Thr Ala Pro Pro Val His Asn Val
1               5


<210>  12
<211>  20
<212>  DNA
<213>  Unknown

<220>
<223>  bacterial DNA

<400>  12
tccatgacgt tcctgacgtt                                                   20


<210>  13
<211>  15
<212>  PRT
<213>  Mycobacterium tuberculosis

<400>  13

Phe Gln Asp Ala Tyr Asn Ala Ala Gly Gly His Asn Ala Val Phe

1                   5                   10                  15


<210>  14
<211>  15
<212>  PRT
<213>  Artificial sequence

<220>
<223>  Synthetic peptide

<400>  14

Phe Gln Asp Ala Tyr Asn Ala Val His Ala Ala His Ala Val Phe
1               5                   10                  15


<210>  15
<211>  13
<212>  PRT
<213>  Artificial sequence

<220>
<223>  Synthetic peptide


<220>
<221>  MOD_RES
<222>  (1)..(1)
<223>  D-alanine

<220>
<221>  MOD_RES
<222>  (3)..(3)
<223>  cyclohexylalanine

<220>
<221>  MOD_RES
<222>  (13)..(13)
<223>  D-alanine

<400>  15

Ala Lys Ala Val Ala Ala Trp Thr Leu Lys Ala Ala Ala
1               5                   10

## Description

### TECHNICAL FIELD

The present invention relates to a vaccine composition for mucosal administration. More particularly, it relates to a vaccine composition for mucosal administration to induce cellular immunity, comprising (i) an antigen; and (ii) one or more cellular immunity induction promoters.

### BACKGROUND ART

Vaccines are widely used in order to induce immunity into the subject and include those for administering pathogens such as microorganisms or viruses or a part thereof. There is a cancer vaccine for allowing a cellular immunity mechanism to recognize a cancer cell specific antigen and inducing a specific attack of the immune system to cancer cells, which is used as one measure for treating a cancer.

In usual, the invasion of microorganisms and viruses into the bio-body is prevented by skin due to the size thereof, and it is necessary to invasively administrate a vaccine into the bio-body. Accordingly, injections are usually used in order to provide immunity. Injections, however, have problems of pain, fear, an injection scar, and a needle mark and scarring thereof, and have further problems that only a medical worker is allowed to perform such administration; it is technically difficult to perform an intradermal injection having a high immune effect; there is a risk such as an infection accident caused by needlestick by a medical worker; patients are forced to go repeatedly to the hospital when repeated injection is required; and it causes medical wastes such as injection needle which is required to be disposed by a special method. Thus, injection is not necessarily an optimal route of administration.

Subcutaneous injection or intradermal injection is most generally used as the route of administration of a vaccine, but in addition to them, various routes of administration have been tried to induce immunity, for example, transdermal administration (Patent Document 1 and Non-Patent Document 1), buccal administration, transnasal administration, and sublingual administration (Non-Patent Document 2, Patent Document 2 and Patent Document 3).

In order to provide immunity by injection, it is usually used an adjuvant. For example, aluminum salts such as aluminum hydroxide, aluminum phosphate and aluminum chloride, and emulsions including squalene such as MF59 and AS03 are practically used as an adjuvant, and in addition to them, flagellum components, nucleic acids, cytokines, cationic polymers and polypeptides are widely studied as an adjuvant. With respect to an adjuvant to be used for other route than injection such as transdermal administration or transmucosal administration to provide immunity, it has also been studied to use a substance such as aluminum salts (e.g. aluminum hydroxide, aluminum phosphate and aluminum chloride), and toxins (e.g. cholera toxin and heat-labile E. coli toxin), but they have not yet been put into practical use. Most of them are used as an adjuvant for inducing humoral immunity by producing antibodies to prevent infection from viruses or bacteria. On the other hand, as for only cellular immunity induction, a Freund adjuvant, Montanide, GM-CSF, IL-2, IL-12 and IFN-γ have been studied as an adjuvant for injection, but they have still not yet been put into practical use. Besides, in the route of transdermal administration or mucosal administration, there are only a few reports about toxins such as cholera toxin and heat-labile E. coli toxin, and nucleic acids.

### LIST OF DOCUMENTS

[Patent Document 1] US-A-2008/0193487
[Patent Document 2] JP-A-2002-531415
[Patent Document 3] US-A-2008/0112974
[Patent Document 4] JP-A-7-505883
[Patent Document 5] JP-A-2007-529531

[Non-Patent Document 1] Hosoi Akihiro et al., Cancer Research, 68, 3941-3949 (2008)
[Non-Patent Document 2] Zhengrong Cui et al., Pharmaceutical Research, Vol.19, No.7, 947-953 (2002)

### SUMMARY OF THE INVENTION

Mucosal administration has been thought as one measure for solving various problems regarding injection. However, there is little report as to a promoter which can induce cellular immunity by mucosal administration of an antigen. In particular in a case of using a peptide as the antigen, there are no report of cellular immunity induction by mucosal administration. Thus, a sufficient cellular immunity induction effect cannot be obtained in mucosal administration, contrary to the route of injection.

An object of the present invention is to provide a vaccine composition which comprises the cellular immunity induction promoter and is highly effective for cellular immunity induction by mucosal administration.

The present invention provides a vaccine composition which comprises a cellular immunity induction promoter universally usable against various antigens in immunity induction by mucosal administration of an antigen and exerts a high cellular immunity inducing effect in mucosal administration. In one aspect of the present invention, the cellular immunity induction caused by mucosal administration of an antigen is potentiated by using a specific cellular immunity induction promoter together with the antigen. Specifically, when one or more cellular immunity induction promoters selected from the group consisting of a TLR ligand, a cyclic dinucleotide, a helper peptide and an immunomodulatory small molecule drug are used in a vaccine composition for mucosal administration, a high cellular immunity inducing effect can be obtained in mucosal administration.

The present invention provides the aspects listed below.
(1) A vaccine composition for mucosal administration to induce cellular immunity, comprising (i) an antigen; and (ii) one or more cellular immunity induction promoters selected from the group consisting of a TLR ligand, a cyclic dinucleotide, a helper peptide and an immunomodulatory small molecule drug;
(2) The vaccine composition according to (1), wherein the cellular immunity induction promoter is a TLR ligand;
(3) The vaccine composition according to (1), wherein the cellular immunity induction promoter is a cyclic dinucleotide;
(4) The vaccine composition according to (1), wherein the Cellular immunity induction promoter is an immunomodulatory small molecule drug;
(5) The vaccine composition according to (1), wherein the cellular immunity induction promoter is a helper peptide;
(6) The vaccine composition according to (1), wherein the cellular immunity induction promoter is a combination of one or more substances selected from the group consisting of a TLR ligand, a cyclic dinucleotide and an immunomodulatory small molecule drug, and a helper peptide; and
(7) The vaccine composition according to any one of (1) to (6), wherein the antigen is a peptide selected from the group consisting of survivin 2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, IPEP87 peptide and/or modified IPEP87 peptide, PR1 peptide and/or modified PR1 peptide, HER2/neu_A02 peptide and/or modified HER2/neu_A02 peptide, MAGE3_A02 peptide and/or modified MAGE3_A02 peptide, HBVenv peptide and/or modified HBVenv peptide, and MUC1 peptide and/or modified MUC1 peptide.
   In another aspect, the vaccine composition of the present invention can be used for the treatment or prevention of diseases. Therefore, the present invention also provides the embodiments listed below.
(8) A method for treating or preventing a cancer comprising mucosally administrating to a subject a therapeutically effective amount of (i) a cancer antigen, and (ii) one or more cellular immunity induction promoters selected from the group consisting of a TLR ligand, a cyclic dinucleotide, a helper peptide and an immunomodulatory small molecule drug;
(9) The method according to (8), wherein the cancer antigen is a cancer antigen peptide selected from the group consisting of survivin 2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, PR1 peptide and/or modified PR1 peptide, HER2/neu_A02 peptide and/or modified HER2/neu_A02 peptide, MAGE3_A02 peptide and/or modified MAGE3_A02 peptide, and MUC1 peptide and/or modified MUC1 peptide;
(10) A method for treating or preventing a viral disease comprising mucosally administrating to a subject a therapeutically effective amount of (i) a virus antigen, and (ii) one or more cellular immunity induction promoters selected from the group consisting of a TLR ligand, a cyclic dinucleotide, a helper peptide and an immunomodulatory small molecule drug; and
(11) The method according to (10), wherein the virus antigen is a peptide selected from the group consisting of IPEP87 peptide and/or modified IPEP87 peptide, and HBVenv peptide and/or modified HBVenv peptide.
   In another aspect, the present invention provides a TLR ligand, a cyclic dinucleotide, a helper peptide, an immunomodulatory small molecule drug, or a mixture of two or more thereof, for use as a cellular immunity induction promoter for mucosal administration against an antigen. The present invention also provides the following aspect:
(12) A TLR ligand, a cyclic dinucleotide, a helper peptide, an immunomodulatory small molecule drug, or a combination of two or more thereof, for use as a cellular immunity induction promoter in cellular immunity induction by mucosal administration of an antigen.
   The present invention also provides the following embodiments:
(13) A method of inducing cellular immunity, comprising mucosally administering to a subject (i) an antigen and (ii) one or more cellular immunity induction promoters selected from the group consisting of TLR ligand, a cyclic dinucleotide, a helper peptide and an immunomodulatory small molecule drug;
(14) TLR ligand, cyclic dinucleotide, helper peptide, immunomodulatory small molecule drug or a combination of two or more kinds of them, for use in promoting the induction of cellular immunity by the mucosal administration of an antigen;
(15) A combination of (i) an antigen and (ii) one or more cellular immunity induction promoters selected from the group consisting of TLR ligand, a cyclic dinucleotide, a helper peptide and an immunomodulatory small molecule drug, for use in inducing cellular immunity by the mucosal administration of an antigen;
(16) A combination of (i) a cancer antigen and (ii) one or more cellular immunity induction promoters selected from the group consisting of TLR ligand, a cyclic dinucleotide, a helper peptide and an immunomodulatory small molecule drug for use in treating or preventing a cancer, wherein the combination is mucosally administered to a subject;
(17) A combination of (i) a virus antigen and (ii) one or more cellular immunity induction promoters selected from the group consisting of TLR ligand, a cyclic dinucleotide, a helper peptide and an immunomodulatory small molecule drug for use in treating or preventing a viral disease, wherein the combination is mucosally administered to a subject;
(18) Use of (i) an antigen and (ii) one or more cellular immunity induction promoters selected from the group consisting of TLR ligand, a cyclic dinucleotide, a helper peptide and an immunomodulatory small molecule drug, for the manufacture of a vaccine composition for mucosal administration intended for the induction of cellular immunity;
(19) Use of (i) a cancer antigen and (ii) one or more cellular immunity induction promoters selected from the group consisting of TLR ligand, a cyclic dinucleotide, a helper peptide and an immunomodulatory small molecule drug, for the manufacture of a vaccine composition for mucosal administration intended for the treatment or prevention of a cancer; and
(20) Use of (i) a virus antigen and (ii) one or more cellular immunity induction promoters selected from the group consisting of TLR ligand, a cyclic dinucleotide, a helper peptide and an immunomodulatory small molecule drug, for the manufacture of a vaccine composition for mucosal administration intended for the treatment or prevention of a viral disease.

The vaccine composition of the present invention can be mucosally administered (in particular, transnasally and through oral mucosal membranes including a sublingual mucous membrane), and thus it has advantages of excellent compliance, for example, noninvasive administration, painlessness, and release from fear of injection, and has further advantages that the composition can be administered by a patient himself/herself because of ease of administration; a risk of an infection accident caused by needlestick by a medical worker can be avoided; the frequency of hospital visit when repeated administrations are performed can be reduced, which contributes to improve the life quality of the patient; and further medical wastes such as an injection needle is not generated. In addition, the vaccine composition of the present invention has also an advantage that the effect as the vaccine is remarkably improved as compared with the case of single administration of an antigen. Furthermore, the vaccine composition of the present invention has also an advantage that the mucosal administration thereof can induce stronger immunity than the injection administration.

### DETAILED DESCRIPTION OF THE INVENTION

In order to more easily understand the present invention, the terms as used herein are defined below. The terms not defined herein have meanings generally understood by those skilled in the art, particularly in the fields of medical science, pharmacy, immunology, cell biology, biochemistry, and polymer chemistry, unless the context requires otherwise.

### I. Definition

The term "antigen" as used herein means any substance capable of inducing an immune response, for example, proteins and peptides. In the mucosal administration, in which the antigen shall be permeable through the mucous membrane, it is preferable to use an antigen having a small molecular weight, for example, a peptide having about 8 to about 12 amino acids. Examples of peptide antigens to be used in the present invention include the following peptides: survivin-2B peptide, GPC3 peptide, HER2/neu_A24 peptide, MAGE3_A24 peptide, IPEP87 peptide, PR1 peptide, HER2/neu_A02 peptide, MAGE3_A02 peptide, HBVenv peptide, HER2/neu E75 peptide, and MUC1 peptide. In one embodiment, one or more peptides selected from the group consisting of HER2/neu E75 peptide for cancer vaccine applications, modified HER2/neu E75 peptide for cancer vaccine applications, WT1 peptide for cancer vaccine applications, and modified WT1 peptide for cancer vaccine applications are excluded from the antigen to be used in the vaccine composition of the present invention. In one embodiment, one or more peptides selected from the group consisting of HER2/neu E75 peptide for cancer vaccine applications and a modified HER2/neu E75 peptide for cancer vaccine applications are excluded from the antigen to be used in the vaccine composition of the present invention.

The term "survivin-2B peptide" as used herein means a peptide derived from a cancer gene product, survivin, consisting of Ala Tyr Ala Cys Asn Thr Ser Thr Leu (SEQ NO: 1).

The term "GPC3 peptide" as used herein means a peptide derived from a cancer gene product, GPC3, consisting of Glu Tyr Ile Leu Ser Leu Glu Glu Leu (SEQ NO: 2).

The term "HER2/neu_A24 peptide" as used herein means an HLA-A24-restricted peptide derived from a cancer gene product, HER2/neu, consisting of Thr Tyr Leu Pro Thr Asn Ala Ser Leu (SEQ NO: 3).

The term "MAGE3_A24 peptide" as used herein means an HLA-A24-restricted peptide derived from a cancer gene product, MAGE3, consisting of Ile Met Pro Lys Ala Gly Leu Leu Ile (SEQ NO: 4).

The term "IPEP87 peptide" as used herein means a peptide derived from hepatitis C virus (HCV) protein, consisting of Asp Leu Met Gly Tyr Ile Pro Ala Val (SEQ NO: 5).

The term "PR1 peptide" as used herein means a peptide derived from a cancer gene product, proteinase-3, consisting of Val Leu Gln Glu Leu Asn Val Thr Val (SEQ NO: 6).

The term "HER2/neu_A02 peptide" as used herein means an HLA-A02-restricted peptide derived from a cancer gene product, HER2/neu, consisting of Lys Val Phe Gly Ser Leu Ala Phe Val (SEQ NO: 7).

The term "MAGE3_A02 peptide" as used herein means an HLA-A02-restricted peptide derived from a cancer gene product, MAGE3, consisting of Lys Val Ala Glu Ile Val His Phe Leu (SEQ NO: 8).

The term "HBVenv peptide" as used herein means a peptide derived from hepatitis B virus (HBV) protein, consisting of Trp Leu Ser Leu Leu Val Pro Phe Val (SEQ NO: 9).

The term "HER2/neu E75 peptide" as used herein means a peptide derived from a product of cancer gene, HER2/neu (HER2 protein), consisting of Lys Ile Phe Gly Ser Leu Ala Phe Leu (SEQ NO: 10).

The term "MUC1 peptide" as used herein means a peptide derived from MUC1 protein, which is a glycoprotein highly expressed on many cancer cells, consisting of Ser Thr Ala Pro Pro Val His Asn Val (SEQ NO: 11).

The term "WT1 peptide" as used herein means a partial peptide consisting of about 8 to about 15, preferably about 8 to about 12, amino acids. The WT1 peptide is a peptide obtained by fragmenting WT1 protein which is a product of a cancer gene, WT1 (Wilm's tumor), and include Db126 peptide and Db235 peptide (both are described in Japanese Patent No. 4422903). In addition, a partial peptide of WT1 product disclosed in WO 2000/06602, a WR1-derived HLA-A26 binding cancer antigen peptide described in WO 2005/095598, an HLA-A* 3303-restricted WT1 peptide described in WO 2007/097358, and HLA-A* 1101-restricted WT1 peptide described in WO 2008/081701 are also included in the "WT1 peptide" of the present invention.

The term "modified XX peptide" (XX is a name of arbitrary peptide) as used herein means a modified peptide in which all or a part of amino acids in a XX peptide are substituted or modified.

Examples of the modified XX peptide include:
(a) a peptide including an amino acid sequence in which one to several, for example, 1, 2, 3, 4 or 5 amino acids are substituted, deleted or added in the amino acid sequence of the XX peptide; and
(b) a peptide including an amino acid sequence in which all or a part of amino acids, for example, one to plural amino acids, such as 1, 2, 3, 4, 5, 6, 7, 8, 9, or 10 amino acids are modified in the amino acid sequence of the XX peptide.

Examples of the "modification" in the amino acid which may occur in the modified XX peptide include, while not limited thereto, aliphatic chain addition modification such as acetylation, alkylation such as methylation, glycosylation, hydroxylation, carboxylation, aldehydation, phosphorylation, sulfonylation, formylation, modification by addition of an aliphatic chain such as myristoylation, palmitoylation or stearoylation, octanoylation, esterification, amidation, deamidation, modification by disulfide bond formation such as cystine modification, glutathione modification or thioglycolic acid modification, glycation, ubiquitination, succinimide formation, glutamylation, prenylation, and the like. The modified XX peptide may include a combination of one or more amino acids substituted, deleted or added with one or more amino acids modification.

In a preferable aspect, the antigen contained in the vaccine composition for mucosal administration of the present invention is a peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, IPEP87 peptide and/or modified IPEP87 peptide, PR1 peptide and/or modified PR1 peptide, HER2/neu_A02 peptide and/or modified HER2/neu_A02 peptide, MAGE3_A02 peptide and/or modified MAGE3_A02 peptide, HBVenv peptide and/or modified HBVenv peptide, and MUC1 peptide and/or modified MUC1 peptide. Alternatively, HER2/neu E75 peptide and/or modified HER2/neu E75 peptide may be used as the antigen.

The peptides listed above can be in free form or in the form of any pharmacologically acceptable salt thereof, for example, acid salts (acetate, TFA salt, hydrochloride, sulfate, phosphate, lactate, tartrate, maleate, fumarate, oxalate, hydrobromide, succinate, nitrate, malate, citrate, oleate, palmitate, propionate, formate, benzoate, picrate, benzenesulfonate, dodecylsulfate, methanesulfonate, p-toluenesulfonate, glutarate, various amino acid salts, and the like), metal salts (alkali metal salts (e.g. sodium salt and potassium salt), alkaline earth metal salts (e.g. calcium salt and magnesium salt), aluminum salts, and the like), and amine salts (triethylamine salt, benzylamine salt, diethanolamine salt, t-butylamine salt, dicyclohexylamine salt, arginine salt, dimethylammonium salt, ammonium salt, and the like). The pharmacologically acceptable salt is preferably acetate and a TFA salt. The peptides described above, which can be used as the antigen in the present invention, may be synthesized or produced by a well-known method, followed by isolation and purification.

The term "cellular immunity induction promoter" as used herein means any substance which can more enhance immune response induced by an antigen, which is administered together with the substance, as compared with the immune response induced by the antigen without the substance. The cellular immunity induction promoter may include substances specified in the specification of the present invention, though it is not limited by the action mechanism by which cellular immunity induction is promoted.

The term "TLR ligand" as used herein means a ligand of a Toll-like receptor (TLR), and include, for example, ligands of TLR1 to TLR9. Examples of the TLR ligand include a ligand of a heterodimer of TLR1 and TLR2 (TLR1/2 ligand), a ligand of a heterodimer of TLR2 and TLR6 (TLR2/6 ligand), a TLR2 and Dectin 1 ligand, a TLR3 ligand, a TLR4 ligand, a TLR5 ligand, a TLR7 and/or TLR8 ligand, a TLR9 ligand, and the like. Any of them can be used as the cellular immunity induction promoter in the present invention. In a preferable aspect of the present invention, the TLR ligand is one selected from the group consisting of a TLR1/2 ligand, a TLR3 ligand, a TLR4 ligand, and a TLR7 and/or TLR8 ligand.

The term "TLR1/2 ligand" as used herein means a ligand of a heterodimer of Toll-like receptor (TLR) 1 and Toll-like receptor (TLR) 2. Examples include triacylated lipoprotein derived from a cell wall of bacterium and a salt thereof, which may be an extract, a product or a synthetic product, but is not limited thereto.

In a preferable aspect of the present invention, the TLR1/2 ligand is Pam₃CSK₄. Pam₃CSK₄ has the formula:

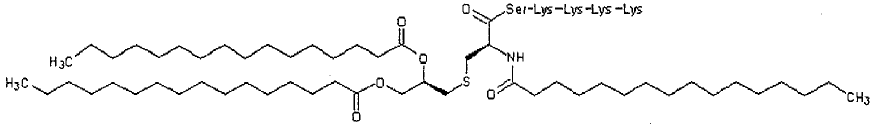

The term "TLR2 ligand" as used herein means TLR2 in the heterodimer of Toll-like receptor (TLR) 1 and Toll-like receptor (TLR) 2, TLR2 in the heterodimer of Toll-like receptor (TLR) 6 and Toll-like receptor (TLR) 2, and a ligand acting with both of them. The TLR2 ligand includes, but is not limited to, for example, bacterial cell wall-derived lipoteichoic acid, and peptidoglycan and a salt thereof, which may be an extract, a product or a synthetic product. In a preferable aspect of the present invention, the TLR2 ligand is peptidoglycan (PGN).

The term "TLR2 and Dectin 1 ligand" as used herein means a ligand of Toll-like receptor (TLR) 2 and β1,3-glucan receptor (Dectin 1), and includes, for example, a β1,3-glucan derived from a cell wall of fungus, and a salt thereof, which may be an extract, a product, or a synthetic product, but is not limited thereto. In a preferable aspect of the present invention, the TLR2 and Dectin 1 ligand is Zymosan derived from a cell wall of yeast.

The term "TLR3 ligand" as used herein means a ligand of Toll-like receptor (TLR) 3. Examples include double stranded RNA (dsRNA) derived from virus, and a salt thereof, which may be an extract, a product, or a synthetic product, but is not limited thereto. In a preferable aspect of the present invention, the TLR3 ligand is polyinosinic-polycytidylic acid (Poly (I:C)) which is a synthetic product, and/or a salt thereof.

The term "TLR4 ligand" as used herein means a ligand of Toll-like receptor (TLR) 4. Examples includes, but is not limited to, lipopolysaccharide (LPS) derived from bacteria or plant, in particular, lipid A derivatives such as monophosphoryl lipid A, 3-deacylated monophosphoryl lipid A (3D-MPL), OM 174, OM 294 DP or OM 197 MP-Ac DP; alkyl glucosaminide phosphate (AGP), for example, AGP disclosed in WO 9850399 or US 6303347, or a salt of AGP disclosed in US 6764840, lipopolysaccharide derived from Pantoea bacterium, glucopyranosyl lipid, and sodium hyaluronate.

In a preferable aspect of the present invention, the TLR4 ligand is preferably a lipopolysaccharide derived from the genus Acetobacter (for example, Acetobacter aceti, Acetobacter xylinum, and Acetobacter orientalis), the genus Zymomonas (for example, Zymomonas mobilis, and the like), the genus Xanthomonas (for example, Xanthomonas campestris), the genus Enterobacter (for example, Enterobacter cloacae), or the genus Pantoea (for example, Pantoea agglomerans). It is possible to use the extract derived from the lipopolysaccharide or purified lipopolysaccharide as it is. In addition, for example, lipopolysaccharide (IP-PA1) derived from Pantoea agglomerans is available from Funakoshi Corporation. In a preferable aspect of the present invention, the TLR4 ligand is lipopolysaccharide derived from Pantoea bacterium, glucopyranosyl lipid and/or sodium hyaluronate.

The term "TLR7 and/or TLR8 ligand" as used herein means a ligand of Toll-like receptor (TLR) 7 and/or TLR 8. It includes, but is not limited to, for example, single stranded RNA, imiquimod, resiquimod (R848), TLR7-II, and other compounds such as loxoribine and bropirimine.

In a preferable aspect of the present invention, the TLR7 and/or TLR8 ligand is imiquimod. The imiquimod is 1-(2-methylpropyl)-1H-imidazo[4,5-c]quinolin-4-amine of the formula: 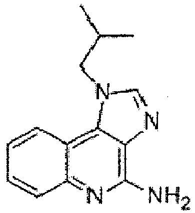 whose characteristics and production method are described, for example, in JP-A-7-505883 (Patent Document 4).

In another preferable aspect, the TLR7 and/or TLR8 ligand is resiquimod. The resiquimod is 4-amino-2-(ethoxymethyl)-α,α-dimethyl-1H-imidazo[4,5-c]quinolin-a-ethanol of the formula:

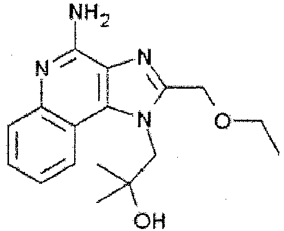

In another preferable aspect, the TLR7 and/or TLR8 ligand is TLR7-II. The TLR7-II is represented by the formula:

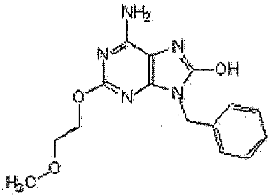

In another preferable aspect, the TLR7 and/or TLR8 ligand is bropirimine. The bropirimine is represented by the formula:

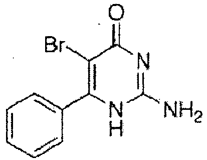

The term "TLR9 ligand" as used herein means a ligand of Toll-like receptor (TLR) 9, and includes, for example, ODN 1826. The TLR9 ligand used in the present invention may be an extract, a product, or a synthetic product, but is not limited thereto. In a preferable aspect of the present invention, the TLR9 ligand is ODN 1826.

ODN 1826 is oligodeoxynucleotide consisting of the following sequence (SEQ NO: 12):
5' - t c c a t g a c g t t c c t g a c g t t - 3'

The term "TLR2/6 ligand" as used herein means a ligand of a heterodimer of Toll-like receptor (TLR) 2 and Toll-like receptor (TLR) 6, and includes, for example, a diacylated lipoprotein derived from a cell wall of mycoplasma, and a salt thereof, which may be an extract, a product, or a synthetic product, but is not limited thereto. In a preferable aspect of the present invention, the TLR2/6 ligand is Pam₂ CSK₄, MALP-2 and/or FSL-1.

Pam₂ CSK₄ is represented by the following formula:

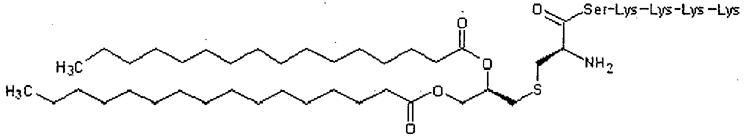

FSL-1 is represented by the following formula:

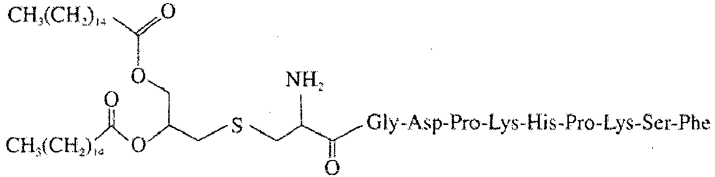

The term "TLR5 ligand" as used herein means a ligand of Toll-like receptor (TLR) 5, and includes, for example, flagellin. The TLR5 ligand used in the present invention may be an extract, a product, or a synthetic product, but is not limited thereto. In a preferable aspect of the present invention, the TLR5 ligand is flagellin.

The Toll-like receptor (TLR) is a family of type I transmembrane proteins, which initiates a congenital immune response in a specific cytokine, a specific chemokine and a growth factor participate, by in vivo activation thereof. All TLRs can activate a certain intracellular signal transmission molecule, for example, a nuclear factor κB (NF-κB) or a mitogen-activated protein kinase (MAP kinase) or the like, while a specific population of a cytokine and a chemokine which are released seems to be inherent in each TLR. TLRs 3, 7, 8 and 9 include a subfamily of TLR which is present in an endosome fraction or a lysosome fraction of an immune cell (e.g. dendritic cells and monocytes). Specifically, TLR3 is expressed by a wide range of cells such as a dendritic cell and a fibroblast; TLR7 is expressed by a plasma-cell like dendritic cell and is expressed by a monocyte to a lesser extent; TLR8 is expressed by a monocyte, as well as a monocyte-derived dendritic cell and a myeloid dendritic cell; and TLR9 is expressed by a plasma-cell like dendritic cell. This subfamily mediates recognition of microorganism nucleic acid (single stranded RNA, double stranded RNA, single stranded DNA, and the like). Agonists of TLR3, TLR7 and/or TLR8, or TLR9 stimulate production of various inflammatory cytokines (which include, for example, interleukin-6, interleukin-12, TNF-α, and interferon-γ). Such agonists also promote increase in expression of a co-stimulator molecule (for example, CD40, CD80, and CD86), a major histocompatibility complex molecule, and a chemokine receptor. Type I interferons (IFN-α and IFN-β) are also produced by a cell upon activation with TLR7 and/or TLR8 agonists.

The term "cyclic dinucleotide" as used herein means a molecule in which two OH groups of a sugar part of two nucleotides produce an ester for such same phosphoric acid molecule and thereby nucleotides are cyclized and an analogs thereof, and includes, for example, cyclic diAMP (c-di-AMP), cyclic diGMP (c-di-GMP), c-dGpGp, c-dGpdGp, c-GpAp, c-GpCp, c-GpUp, and the like, but is not limited thereto. The cyclic dinucleotide activates a dendritic cell or a T cell. Further examples of the cyclic dinucleotide, use thereof as an adjuvant, and a process for production thereof are described in JP-A-2007-529531 (Patent Document 5). In a preferable aspect of the present invention, the cyclic dinucleotide is cyclic diGMP and/or cyclic diAMP. The cyclic diGMP has the formula: 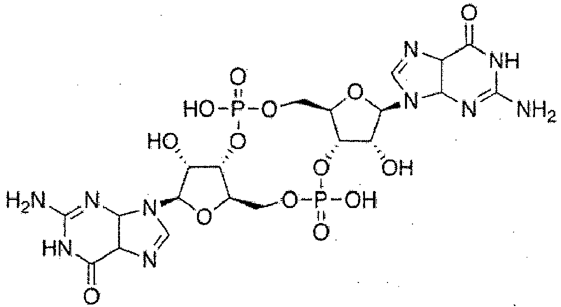 and a process for synthesis thereof is described in Kawai et al., Nucleic Acids Research Suppl.3:103-4.

The terms "helper peptide" as used herein means any peptide which activates a helper T cell, and includes, for example, tubercle bacillus-derived helper peptide, measles virus-derived helper peptide, hepatitis B virus-derived helper peptide, hepatitis C virus-derived helper peptide, Chlamydia trachomatis-derived helper peptide, Plasmodium falciparum sporozoite-derived helper peptide, keyhole limpet haemocyanin-derived helper peptide, tetanus toxin-derived helper peptide, pertussis toxin-derived helper peptide, diphtheria toxin-derived helper peptide, cancer cell-derived helper peptide (for example, IMA-MMP-001 helper peptide, CEA-006 helper peptide, MMP-001 helper peptide, TGFBI-004 helper peptide, HER-2/neu (aa776-790) helper peptide, AE36 helper peptide, AE37 helper peptide, MET-005 helper peptide, and BIR-002 helper peptide), and universal helper analogs (for example, PADRE).

In the present invention, in place of or in combination with the helper peptide described above, a peptide in which all or a part of the amino acids in the helper peptide are modified by substitution, modification or the like (hereinafter, referred to as a "modified helper peptide") can also be used.

Examples of the modified helper peptide include:
(a) a peptide consisting of an amino acid sequence in which one to several, for example, 1, 2, 3, 4 or 5 amino acids are substituted, deleted, or added in the amino acid sequence of the original helper peptide; and
(b) a peptide consisting of an amino acid sequence in which all or a part of amino acids, for example, 1 to several, e.g., 1, 2, 3, 4, 5, 6, 7, 8, 9, 10, 11, 12, 13, 14 or 15 amino acids are modified in the amino acid sequence of the original helper peptide.

Examples of the "modification" in the amino acid which may occur in the modified helper peptide include, but is not limited thereto, aliphatic chain addition modification such as acetylation, alkylation such as methylation, glycosylation, hydroxylation, carboxylation, aldehydation, phosphorylation, sulfonylation, formylation, addition of fatty acid such as myristoylation, palmitoylation, and stearoylation, octanoylation, esterification, amidation, deamidation, modification by disulfide bond formation such as cystine modification, glutathione modification or thioglycolic acid modification, glycation, ubiquitination, succinimide formation, glutamylation, prenylation, and the like. The modified helper peptide may include a combination of substitution, deletion or addition of one or more amino acids.

In a preferable aspect of the present invention, the helper peptide consists of 10 to 18 amino acids, preferably 12 to 16 amino acids, more preferably 13 to 15 amino acids. In a preferable aspect of the present invention, the helper peptide is Peptide-25, modified Peptide-25, or PADRE. One example of the modified Peptide-25 is Peptide-25B. The Peptide-25 is a peptide of 15 amino acids consisting of Phe Gln Asp Ala Tyr Asn Ala Ala Gly Gly His Asn Ala Val Phe (SEQ NO: 13) which corresponds to amino acid residues 240 to 254 of Ag85B which is one of the major proteins secreted by human tubercle bacillus (Mycobacterium tuberculosis). The Peptide-25B is one example of the modified Peptide-25 obtained by modification of a part of amino acids in the Peptide-25 for increasing in immunostimulation effect thereof, and is a peptide of 15 amino acids consisting of Phe Gln Asp Ala Tyr Asn Ala Val His Ala Ala His Ala Val Phe (SEQ NO: 14). PADRE is a peptide of 13 amino acids, consisting of D-Ala Lys cyclohexyl-Ala Val Ala Ala Trp Thr Leu Lys Ala Ala D-Ala (SEQ NO: 15).

The term "immunomodulatory small molecule drug" as used herein means a substance which activates or inhibits immune cells such as a T cell, an NK cell and a macrophage, provided that the TLR ligand, the cyclic dinucleotide and the helper peptide described above are excluded. Examples of the immunomodulatory small molecule drug include bestatin, pidotimod, levamisole, golotimod, forphenicinol, and their derivatives, and pharmacologically acceptable salts thereof. For example, the pharmacologically acceptable salt of levamisole includes levamisole hydrochloride and the like.

Bestatin is represented by the formula:

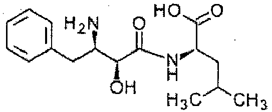

Pidotimod is represented by the formula:

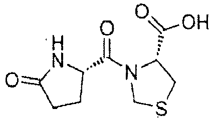

Levamisole hydrochloride is represented by the formula:

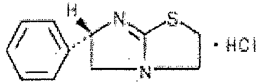

In the present invention, the immunomodulatory small molecule drug is usually a compound having a molecular weight of less than 1000, preferably less than 500. In a preferable aspect of the present invention, the immunomodulatory small molecule drug is one or more compounds selected from the group consisting of bestatin, pidotimod and levamisole hydrochloride.

In one aspect of the present invention, it has been found that a TLR ligand, a cyclic dinucleotide, a helper peptide, or an immunomodulatory small molecule drug is preferable for the cellular immunity induction, when a desired antigen is mucosally administered. Accordingly, in one aspect, the cellular immunity induction promoter of the present invention comprises one or more substances selected from them. In a particularly preferable aspect of the present invention, the cellular immunity induction promoter is a combination of one or more substances selected from a TLR ligand, a cyclic dinucleotide and an immunomodulatory small molecule drug, with a helper peptide. Various methods have been developed as a method for quantitatively measuring the cellular immunity induction, and one or more of them, for example, the ELISPOT method, described in Examples, may be used.

In a preferable aspect, the cellular immunity induction promoter contained in the vaccine composition for mucosal administration of the present invention is one or more members selected from the group consisting of a TLR ligand, a cyclic dinucleotide, a helper peptide and an immunomodulatory small molecule drug, more preferably is a combination of one or more substances selected from the group consisting of a TLR ligand, a cyclic dinucleotide and an immunomodulatory small molecule drug, with a helper peptide. In a particularly preferable aspect, the cellular immunity induction promoter is a combination of one or more substances selected from the group consisting of a TLR1/2 ligand, a TLR3 ligand, a TLR4 ligand, a TLR7 and/or TLR8 ligand, a cyclic diGMP and levamisole hydrochloride, with a helper peptide.

The term "non-invasive administration" as used herein means an administration without positively providing physical irritation and/or chemical irritation, preferably without physical irritation to the mucous membrane (for example, a treatment of releasing a mucous membrane, a treatment of giving damage to a mucous membrane, or a treatment of perforating a mucous membrane).

The term "cancer" as used herein means a cancer associated with abnormal expression of a cancer gene, for example, a cancer with over-expression of cancer gene, for example, hematopoietic tumor or solid cancer. Examples of the cancer gene include survivin gene, GPC3 gene, HER2/neu gene, MAGE3 gene, MAGE A1 gene, MAGE A3/A6 gene, MAGE A4 gene, MAGE12 gene, proteinase-3 gene, AFP gene, CA-125 gene, CD44 gene, CEA gene, c-Kit gene, c-met gene, c-myc gene, L-myc gene, COX2 gene, cyclinD1 gene, Cytokeratin-7 gene, Cytokeratin-19 gene, Cytokeratin-20 gene, E2F1 gene, E2F3 gene, EGFR gene, Gli1 gene, hCGβ gene, HIF-1α gene, HnRNP A2/B1 gene, hTERT gene, MDM gene, MDR-1 gene, MMP-2 gene, MMP-9 gene, Muc-1 gene, Muc-4 gene, Muc-7 gene, NSE gene, ProGRP gene, PSA gene, RCAS1 gene, SCC gene, Thymoglobulin gene, VEGF-A gene, VEGF-A gene, and the like. The cancer associated with abnormal expression of survivin gene includes malignant lymphoma, bladder cancer, lung cancer, and colon cancer, but is not limited thereto. The cancer associated with abnormal expression of GPC3 gene includes liver cancer, bile duct cancer, and gastric cancer, but is not limited thereto. The cancer associated with abnormal expression of HER2/neu gene includes breast cancer, gastric cancer, ovarian cancer, uterine cancer, bladder cancer, non-small-cell lung cancer, prostate cancer, and the like, but is not limited thereto. The cancer associated with abnormal expression of MAGE3 gene includes melanoma, lung cancer, head and neck cancer, bladder cancer, gastric cancer, esophageal cancer, liver cancer, and the like, but is not limited thereto. The cancer associated with abnormal expression of proteinase-3 gene includes acute myelogenous leukemia, pancreatic cancer, and the like, but is not limited thereto.

The terms "abnormal expression of a gene" as used herein means that a gene expression level in cells is increased or decreased remarkably, for example two times or more, or four times or more, as compared with other cells of the same tissue. The term "over-expression" means that the abnormal expression is an increase in the expression level. The expression level of a gene can be easily measured using any method well-known in the art.

The term "subject" as used herein means any animal whose immune response can be induced by the administration of the vaccine composition for mucosal administration in a practical stage, and includes typically a mammal including human, mouse, rat, dog, cat, rabbit, horse, cow, sheep, pig, goat, monkey, and chimpanzee. A particularly preferable subject is human.

The term "model animal for immunological evaluation" as used herein means a model animal for evaluating the immunity induction property of the vaccine composition for mucosal administration, specifically a model animal for evaluating the cellular immunity induction level. In view of compatibility between the antigen in the vaccine composition and MHC class 1 molecule in an animal, an animal in which cellular immunity induction caused by the antigen in the vaccine composition can be evaluated is used as the model animal for immunological evaluation. For example, in case of a vaccine composition containing HLA-A* type 24 MHC-restricted class 1 peptide, the property is evaluated with BALB/c mice. In case of a vaccine composition containing HLA-A* type 02 MHC-restricted peptide, the property is evaluated with gene modified mouse in which cellular immunity induction by HLA-A* type 02 MHC-restricted peptide can be evaluated. In case of a vaccine composition containing other HLA type MHC-restricted peptide, the property is evaluated in an animal in which cellular immunity induction by the HLA type MHC-restricted peptide can be evaluated. In case of a vaccine composition containing a protein antigen, the property is evaluated in an animal having MHC compatible with a class 1 epitope which is intended to provide immunity induction among class 1 epitopes contained in the amino acid sequence of the protein antigen.

The term "cancer antigen" as used herein means a substance such as protein or peptide capable of specifically expressing tumor cells or cancer cells, and of inducing an immune response.

The term "cancer antigen peptide" as used herein means a partial peptide derived from a cancer antigen protein, which can induce an immune response. The cancer antigen peptide is usually generated by decomposition of a cancer antigen protein, which is a cancer gene product, in the cancer cell, and is represented on the surface of the cancer cell by MHC class I molecule. The cancer antigen peptide used in a cancer vaccine formulation may be an endogenous cancer antigen peptide which is isolated and purified from a cancer cell, or may be a synthetic peptide having the same amino acid sequence as that of the endogenous cancer antigen peptide. In a preferable aspect of the present invention, the cancer antigen peptide to be used for inducing immunity is, for example, an endogenous or synthetic cancer antigen peptide selected from the group consisting of survivin-2B peptide and/or modified survivin-2B peptide, GPC3 peptide and/or modified GPC3 peptide, HER2/neu_A24 peptide and/or modified HER2/neu_A24 peptide, MAGE3_A24 peptide and/or modified MAGE3_A24 peptide, PR1 peptide and/or modified PR1 peptide, HER2/neu_A02 peptide and/or modified HER2/neu_A02 peptide, MAGE3_A02 peptide and/or modified MAGE3_A02 peptide, and MUC1 peptide and/or modified MUC1 peptide.

The term "virus antigen" as used herein means a substance derived from a virus or a constituent component thereof, or a substance derived therefrom, which can induce an immune response. Accordingly, a viral disease can be treated or prevented by mucosally administering the virus antigen, preferably together with a cellular immunity induction promoter, to a subject. In a preferable aspect of the present invention, for example, a peptide selected from the group consisting of IPEP87 peptide and/or modified IPEP87 peptide, and HBVenv peptide and/or modified HBVenv peptide can be used as the virus antigen.

The term "viral disease" as used herein means a disease caused by infection and proliferation of virus. Examples include hepatitis A, hepatitis B, hepatitis C, hepatitis D, hepatitis E, cervical cancer, condyloma acuminatum, HIV infection, Chlamydia infection, herpes simplex, and the like.

### II. Vaccine Composition for Mucosal Administration

The vaccine composition for mucosal administration of the present invention exhibits a high cellular immunity induction effect by the mucosal administration of various antigens to a subject.

The term the "composition for mucosal administration" as used herein may be any formulation usually used for mucosal administration such as a sublingual, transnasal, buccal, rectal or vaginal administration, a semi-solid formulation such as a gel formulation (jelly formulation), a cream formulation, an ointment, or a plaster; a liquid formulation; a solid formulation such as a powder formulation, a fine granule formulation, a granule formulation, a film formulation, a tablet formulation, or an orally-disintegrating tablet formulation; a spray formulation for a mucous membrane such as an aerosol formulation; or an inhalating formulation. The classification, definition, characteristics, and production methods thereof are well-known in the art, and see, for example, the Japanese Pharmacopoeia, the 16th edition.

As a solvent for the liquid formulation, for example, an appropriate amount of a solvent such as water, ethanol, glycerol or propylene glycol can be used, and the components can be dispersed or dissolved in the solvent to prepare the liquid formulation.

Examples of the base material for the gel formulation (jelly formulation) to be used as hydrogel base are a carboxyvinyl polymer, a gel base, a fat-free ointment, polyvinyl pyrrolidone, polyvinyl alcohol, sodium polyacrylate, carboxymethylcellulose, starch, xanthan gum, karaya gum, sodium alginate, methylcellulose, hydroxypropyl cellulose, hydroxypropyl methylcellulose phthalate (HPMCP), cellulose acetate phthalate (CAP), carboxymethylethylcellulose (CMEC), ethylcellulose, hydroxyethylcellulose, hydroxypropyl methylcellulose, a carboxyvinyl polymer, tragacanth, gum arabic, tara gum, tamarind seed gum, psyllium seed gum, agar, gel lan gum, glucomannan, locust bean gum, guar gum, carrageenan, dextrin, dextran, amylose, carboxymethylcellulose potassium, carboxymethylcellulose sodium, carboxymethylcellulose calcium, pullulan, chitosan, carboxymethyl starch sodium, Plantago testa, galactomannan, Eudragit, casein, alkyl ester of alginic acid, gelatin, and polyethylene glycol. These base materials can be dissolved in the solvent to prepare a gel formulation having fluidity or formability. The solvent is preferably water, and glycerol and propylene glycol can also be used.

Examples of the base material for the cream formulation include water/oil base materials such as a hydrophilic ointment and vanishing cream; and oil/water base materials such as hydrophilic vaseline, purified lanolin, Aquahole, Eucerin, Neocerin, hydrous lanolin, cold cream, and hydrophilic plastibase. These base materials are added to a fatty solvent or water, and the mixture is stirred at a high speed using a homogenizer to prepare the cream formulation.

Examples of the base material for the film formulation include polyvinyl pyrrolidone, polyvinyl alcohol, sodium polyacrylate, carboxymethyl cellulose, starch, xanthan gum, karaya gum, sodium alginate, methylcellulose, a carboxyvinyl polymer, agar, hydroxypropyl cellulose, hydroxypropyl methylcellulose phthalate (HPMCP), cellulose acetate phthalate (CAP), carboxymethylethylcellulose (CMEC), ethylcellulose, hydroxyethylcellulose, hydroxypropyl methylcellulose, a carboxyvinyl polymer, tragacanth, gum arabic, locust bean gum, guar gum, carrageenan, dextrin, dextran, amylose, carboxymethylcellulose potassium, carboxymethylcellulose sodium, carboxymethylcellulose calcium, pullulan, chitosan, carboxymethyl starch sodium, Plantago testa, galactomannan, an aminoalkyl methacrylate copolymer E, an aminoalkyl methacrylate copolymer RS, methacrylic acid copolymer L, methacrylic acid copolymer LD, methacrylic acid copolymer S, a methylacrylate-methacrylic acid-methyl methacrylate copolymer, an ethyl acrylate-methyl methacrylate copolymer, polyvinyl acetal diethyl aminoacetate, casein, and an alginic acid alkyl ester. These base materials are dissolved in water or a polar organic solvent such as ethanol, the mixture is applied to form a thin film, and the film is dried, whereby the film formulation can be prepared. In one preferable aspect, the vaccine composition for mucosal administration of the present invention is in the form of film formulation.

For preparing the powder formulation, fine granule formulation, granule formulation or tablet formulation, there are used an excipient such as lactose, corn starch or crystalline cellulose, and a binding agent such as hydroxypropyl cellulose and gum arabic, and these additives are mixed in an appropriate amount of a solvent such as water or ethanoland stirred, and followed by granulation, drying and tableting steps. If necessary, a lubricant such as magnesium stearate and a coating agent such as hydroxypropyl cellulose or sucrose may be added.

Examples of the base material for the orally-disintegrating tablet (lyophilization type) include polysaccharides such as gelatin and pullulan. As a forming agent, mannitol, trehalose, sorbitol, glycine, and the like may be used. These additives are dissolved in water, and the mixture is lyophilized after dispensation, whereby the orally-disintegrating tablet (lyophilization-type) can be prepared. In one preferable aspect, the vaccine composition for mucosal administration of the present invention is in the form of orally-disintegrating tablet.

Examples of the aerosol formulation include a liquid formulation, a gel formulation having a high fluidity, a cream formulation, a fine powder such as a powder formulation, as the content thereof. They are dispersed in gas in the form of solid or liquid fine particles using a spraying device, whereby it can be efficiently administered to an administration site such as an oral mucous membrane or nasal mucous membrane.

A ratio between the antigen and the cellular immunity induction promoter in the composition of the present invention is not particularly limited. In one aspect, the composition of the present invention preferably includes the desired antigen in an amount of 0.01 to 40% by weight, more preferably 0.1 to 30% by weight, based on the total weight of the composition. In one aspect, the composition of the present invention preferably contains the cellular immunity induction promoter in an amount of 0.001 to 30% by weight, more preferably 0.01 to 20% by weight, based on the total weight of the composition.

The composition of the present invention, if necessary, may include other additives. The additives can be selected, for example, from an isotonizing agent, an antiseptic/antimicrobial agent, an antioxidant, a solubilizer, a solubilizing aid, a suspending agent, a filler, a pH-controlling agent, a stabilizer, an absorption promoter, a releasing speed controller, a coloring agent, a plasticizer, a cross-linking agent, an adhesive, and a mixture of two or more thereof, which are selected appropriately in view of the compatibility with the main component of the base, the antigen, and the cellular immunity induction promoter, the desired dosage regimen, and the like.

The composition of the present invention can further contain a pharmacologically acceptable acid or a pharmacologically acceptable salt thereof, which is a second cellular immunity induction promoter, to improve immunity induction promoting effect.

The "pharmacologically acceptable acid" as used herein, which can be contained in the composition of the present invention, means an acid which does not provide a harmful effect to a subject to be administered, and does not extinguish the pharmacological activity in the component in the composition. In a preferable aspect of the present invention, the pharmacologically acceptable acid is an organic acid; more preferably an organic compound containing carboxyl group or an organic compound containing sulfonate group; more preferably a saturated or unsaturated straight or branched fatty acid having a saturated straight moiety having 8 to 20 carbon atoms, lactic acid, malic acid, salicylic acid, maleic acid, citric acid, or an organic compound containing sulfonate group; more preferably a saturated or unsaturated straight or branched fatty acid having a saturated straight moiety having 8 to 16 carbon atoms, lactic acid, malic acid, salicylic acid, maleic acid, citric acid, or an organic compound containing sulfonate group; more preferably a fatty acid selected from the group consisting of decanoic acid, lauric acid, myristic acid, isostearic acid, palmitic acid, stearic acid and oleic acid, or lactic acid, salicylic acid, citric acid or methanesulfonic acid.

The "pharmacologically acceptable salt" as used herein, which can be contained in the composition of the present invention, means a salt which does not provide a harmful effect to a subject to be administered, and does not extinguish the pharmacological activity in the component in the composition. The salt includes, but is not limited to, inorganic acid salts (e.g., hydrochloride and phosphate), organic acid salts (e.g., acetate, phthalate, and TFA salt), metal salts (alkali metal salts (e.g., sodium salt and potassium salt), alkaline earth salts (for example, calcium salt and magnesium salt), aluminum salts, and the like), and amine salts (triethylamine salt, benzylamine salt, diethanolamine salt, t-butylamine salt, dicyclohexylamine salt, arginine salt, dimethylammonium salt, ammonium salt, and the like).

The therapeutically effective amount of the antigen can widely vary depending on the severity of a disease, the age and relative health of a subject, and other known factors, and in general, a daily dose of about 0.1 µg to 1 g/kg body weight can provide a satisfactory result. The cellular immunity induction promoter is administered simultaneously or sequentially when the antigen is administered, preferably simultaneously. The effective amount of the cellular immunity induction promoter can widely vary depending on the specific cellular immunity induction promoter to be used, and the presence or absence of another cellular immunity induction promoter, and an amount of 0.01 µg to 1 g/kg body weight can provide a satisfactory result. A daily dose can be administered once, and it may be divided into two or more, for example, two, three, four or five aliquots, and administered. Although the interval between administrations is arbitrary interval, and is appropriately selected depending on the state of a patient, the severity of a disease, and whether the purpose is therapeutic or preventive, from, for example, once a day, once every three days, once a week, once every two weeks, once a month, once every three months, once every six months, once a year, or more. In general, for the therapeutic purpose of a patient actually suffering from a severe disease, the antigen is more frequently administered in a higher dose, and for the preventive purpose of a patient suffering from no disease, the antigen is fewer frequently administered in a lower dose.

The present invention is illustrated more particularly and specifically by the following Examples, but should not be construed to be limited thereto.

### EXAMPLES

### Liquid Formulation for Sublingual Administration

Each liquid formulation having each composition shown in Tables 1 to 9 below was produced and used as an administration sample in mouse immunity experiments. Specifically, 20 parts by weight of an additive (DMSO) and saline as a base material were added to an antigen peptide, a cellular immunity induction promoter, and, if desired, a pharmacologically acceptable acid in amounts set forth in Tables 1 to 9 so that the total amount was 100 parts by weight, and the resultant was mixed to prepare a liquid formulation for sublingual administration.

GPC3 peptide, survivin 2B peptide, HER2/neu_A24 peptide, MAGE3_A24 peptide, IPEP87 peptide, HER2/neu E75 peptide, PR1 peptide, HER2/neu_A02 peptide, MAGE3_A02 peptide, HBVenv peptide, and Peptide-25 were all chemically synthesized and HPLC-purified before use. OVA protein was purchased from Sigma-Aldrich.

Imiquimod was purchased from Tokyo Chemical Industry Co. , Ltd. Cyclic diGMP (c-di-GMP) and cyclic diAMP (c-di-AMP) were purchased from Biolog Life Science Institute. Pam₃ CSK₄ manufactured by InvivoGen, Inc., Poly(I:C)manufactured by InvivoGen, Inc., Pantoea bacterium-derived lipopolysaccharide manufactured by MARCOΦ, glucopyranosyl lipid manufactured by InvivoGen, Inc. (MPLAs), resiquimod (R848) manufactured by InvivoGen, Inc., and levamisole hydrochloride manufactured by MP Biomedical Inc. were used.

### Film Formulation

To 46 parts by weight of D-mannitol (manufactured by Roquette Corporate) and 2.6 parts by weight of polyethylene glycol 400 (manufactured by Wako Pure Chemical Industries, Ltd.) was added 150 parts by weight of purified water, and the mixture was stirred with ultrasonic waves. To the mixture were added 46 parts by weight of hydroxypropyl cellulose (HPC-SSL manufactured by Nippon Soda Co., Ltd.), 5 parts by weight of an antigen peptide (chemically synthesized and HPLC-purified product), 0.3 parts by weight of Peptide-25 (chemically synthesized and HPLC-purified product), and 0.1 parts by weight of a cellular immunity induction promoter other than the helper peptide, and the resultant was thoroughly stirred and mixed. The solution was added dropwise in an amount of 1/100 (2.5 parts by weight) to a polyethylene terephthalate release film, which was air-dried and then dried under reduced pressure to provide 1 part by weight of a film formulation.

### Orally-Disintegrating Tablet

To 20 parts by weight of gelatin (water-soluble gelatin CSF manufactured by Nippi Inc.) and 74.6 parts by weight of D-mannitol was added 500 parts by weight of purified water, and the components were dissolved with stirring. To the solution were added 5 parts by weight of an antigen peptide (chemically synthesized and HPLC-purified product), 0.3 parts by weight of Peptide-25 (chemically synthesized and HPLC-purified product), and 0.1 parts by weight of a cellular immunity induction promoter other than the helper peptide, and they were dissolved. The solution was dispensed into formed aluminum containers, which were subjected to a lyophilization treatment overnight to provide an orally-disintegrating tablet. In mouse immunity experiments, the orally-disintegrating tablet was pulverized, and 10 mg of the tablet powder was weighed and used.

### Mouse Immunity Experiment 1 (Sublingual Administration)

Mouse immunity experiments for the sublingual administration liquid formulation, film formulation and orally-disintegrating tablet were performed. The experiments were performed in accordance with the ELISPOT method. Specifically, in a case where the administration was performed once, the liquid formulation, the film formulation or the orally-disintegrating tablet was administered sublingually to anesthetized mice, and the mice were kept for 2 minutes as they were, and were fed for 6 days. In a case where the administration was performed twice, the same procedure as above was repeated after 6 days of the first administration. After 6 days from the final administration, the spleen was isolated, and the antigen-specific cellular immunity induction level was evaluated in accordance with the ELISPOT method.

### (ELISPOT Method)

A suspension of splenocytes was prepared from the spleen isolated. Splenocytes (3 × 10⁶ cells/well) and an antigen (100 µM for an antigen peptide, 100 µg/mL for an antigen protein) were added to wells of an ELISPOT plate to which anti-mouse IFN-γ antibodies were immobilized, together with a culture medium, and were co-cultured in culture conditions of 37°C and 5% CO₂ for 20 hours, and the number of spots of IFN-γ productive cells (the number of spots/3 × 10⁶ cells) was evaluated.

The results of the immunity experiments are shown in Tables 1 to 9 below, together with the mice used, the dosage, and the number of administrations. The "genetically modified mice" in Tables are genetically modified mice from which the cellular immunity induction owing to HLA-A* 0201 MHC-restricted peptide can be evaluated. For comparison, the results obtained from immunity caused by injection formulations described below (Comparative Examples 2 to 4 and 6 to 8) are also shown in each Table.

**Table 1**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 1 | Liquid formulation | Saline | Survivin 2B(2.5) | None | None | None | 20 µL | twice | BALB/c | 5 |
| Example 1 | Liquid formulation | Saline | Survivin 2B(2.5) | LPS(0.1) | None | None | 20 µL | twice | BALB/c | 25 |
| Example 2 | Liquid formulation | Saline | Survivin 2B(2.5) | None | PEPB (0.3) | None | 20 µL | twice | BALB/c | 15 |
| Example 3 | Liquid formulation | Saline | Survivin 2B(2.5) | LPS(0.1) | PEPB (0.3) | None | 20 µL | twice | BALB/c | 49 |
| Example 4 | Liquid formulation | Saline | Survivin 2B(2.5) | LPS(0.1) | PEPB (0.3) | MA(0.05) | 20 µL | twice | BALB/c | 80 |
| Example 5 | Liquid formulation | Saline | Survivin 2B(2.5) | LPS(0.1) | PEPB (0.3) | Isostearic acid (0.05) | 20 µL | twice | BALB/c | 72 |
| Example 6 | Liquid formulation | Saline | Survivin 2B(2.5) | LPS(0.1) | PEPB (0.3) | Lactic acid (0.05) | 20 µL | twice | BALB/c | 76 |
| Example 7 | Liquid formulation | Saline | Survivin 2B(2.5) | LPS(0.1) | PEPB (0.3) | Citric acid (0.05) | 20 µL | twice | BALB/c | 70 |
| Example 8 | Liquid formulation | Saline | Survivin 2B(2.5) | syn-MPL (0.1) | PEPB (0.3) | None | 20 µL | twice | BALB/c | 40 |
| Example 9 | Liquid formulation | Saline | Survivin 2B(2.5) | Imiquimod (0.3) | PEPB (0.3) | None | 20 µL | twice | BALB/c | 20 |
| Example 10 | Liquid formulation | Saline | Survivin 2B(2.5) | c-di-GMP (0.2) | None | None | 10 µL | once | BALB/c | 254 |
| Example 11 | Liquid formulation | Saline | Survivin 2B(2.5) | c-di-GMP (0.2) | PEPB (0.3) | None | 10 µL | once | BALB/c | 333 |
| Example 12 | Liquid formulation | Saline | Survivin 2B(2.5) | Levamisole HCl (0.5) | PEPB (0.3) | None | 20 µL | twice | BALB/c | 16 |
| Example 13 | Film formulation | HPC/PEG/ mannitol | Survivin 2B(5) | LPS(0.1) | PEPB (0.3) | None | 10 mg | twice | BALB/c | 53 |
| Example 14 | Orally disintegrating tablet | Gelatin/ mannitol | Survivin 2B(5) | LPS(0.1) | PEPB (0.3) | None | 10 mg | twice | BALB/c | 54 |
| Comparative Example 2 | Subcutaneous injection | Saline | Survivin 2B(0.125) | Montanide ISA51VG (50) | | None | 200 µL | once | BALB/c | 313 |

| | | | | | | | | | | |
|---|---|---|---|---|---|---|---|---|---|---|
| Numbers in () are a blending ratio of each component (part by weight). (The same applies to the following Tables.) HPC: Hydroxypropyl cellulose PEG: Polyethylene glycol 400 LPS: Pantoea bacterium-derived lipopolysaccharide (TLR4 ligand) Imiquimod: TLR7 and/or TLR8 ligand syn-MPL: synthetic monophosphoryl lipid A (Glucopyranosyl lipid) (TLR4 ligand) c-di-GMP: cyclic diGMP (cyclic dinucleotide) Levamisole HCl: Levamisole hydrochloride (immunomodulatory small molecule drug) PEPB: Peptide-25B (SEQ No: 14) (helper peptide) MA: Myristic acid | | | | | | | | | | |

**Table 2**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 9 | Liquid formulation | Saline | MAGE3_A02(5) | None | None | None | 20 µL | twice | Genetically modified | 6 |
| Example 15 | Liquid formulation | Saline | MAGE3_A02(5) | None | PEP (0.3) | None | 20 µL | twice | Genetically modified | 15 |
| Example 16 | Liquid formulation | Saline | MAGE3_A02(5) | LPS (0.1) | None | None | 20 µL | twice | Genetically modified | 40 |
| Example 17 | Liquid formulation | Saline | MAGE3_A02(5) | LPS (0.1) | PEP (0.3) | None | 20 µL | twice | Genetically modified | 69 |
| Example 18 | Liquid formulation | Saline | MAGE3_A02 (5) | c-di-GMP (0.2) | None | None | 10 µL | once | Genetically modified | 650 |
| Example 19 | Liquid formulation | Saline | MAGE3_A02 (5) | c-di-GMP (0.2) | PEP (0.3) | None | 10 µL | once | Genetically modified | 892 |

| | | | | | | | | | | |
|---|---|---|---|---|---|---|---|---|---|---|
| PEP: Peptide-25 (SEQ NO: 13) (helper peptide) | | | | | | | | | | |

**Table 3**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Example 20 | Liquid formulation | Saline | HER2/neu_A24 (10) | c-di-GMP (0.2) | PEPB (0.3) | None | 10 µL | once | BALB/c | 69 |

**Table 4**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 10 | Liquid formulation | Saline | IPEP87 (10) | None | None | None | 20 µL | twice | Genetically modified | 4 |
| Example 21 | Liquid formulation | Saline | IPEP87 (10) | None | PEP (0.3) | None | 20 µL | twice | Genetically modified | 10 |
| Example 22 | Liquid formulation | Saline | IPEP87 (10) | LPS (0.1) | None | None | 20 µL | twice | Genetically modified | 25 |
| Example 23 | Liquid formulation | Saline | IPEP87 (10) | LPS (0.1) | PEP(0.3) | None | 20 µL | twice | Genetically modified | 42 |
| Example 24 | Liquid formulation | Saline | IPEP87 (10) | Imiquimod (0.3) | None | None | 20 µL | twice | Genetically modified | 132 |
| Example 25 | Liquid formulation | Saline | IPEP87 (10) | Imiquimod (0.3) | PEP (0.3) | None | 20 µL | twice | Genetically modified | 184 |
| Example 26 | Liquid formulation | Saline | IPEP87 (10) | c-di-GMP (0.2) | None | None | 10 µL | once | Genetically modified | 230 |
| Example 27 | Liquid formulation | Saline | IPEP87 (10) | c-di-GMP (0.2) | PEP (0.3) | None | 10 µL | once | Genetically modified | 287 |

**Table 5**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 11 | Liquid formulation | Saline | PR1 (1.25) | None | None | None | 10 µL | once | Genetically modified | 6 |
| Example 28 | Liquid formulation | Saline | PR1 (1.25) | c-di-GMP (0.2) | None | None | 10 µL | once | Genetically modified | 153 |
| Example 29 | Liquid formulation | Saline | PR1 (1.25) | c-di-GMP (0.2) | PEP (0.3) | None | 10 µL | once | Genetically modified | 244 |
| Comparative Example 3 | Subcutaneous injection | Saline | PR1 (0.125) | Montanide ISA51VG (50) | | None | 200 µL | once | Genetically modified | 144 |

**Table 6**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 12 | Liquid formulation | Saline | HER2/neu_A02 (1.25) | None | None | None | 20 µL | twice | Genetically modified | 3 |
| Example 30 | Liquid formulation | Saline | HER2/neu_A02 (1.25) | LPS (0.1) | None | None | 20 µL | twice | Genetically modified | 15 |
| Example 31 | Liquid formulation | Saline | HER2/neu_A02 (1.25) | LPS (0.1) | PEP (0.3) | None | 20 µL | twice | Genetically modified | 21 |
| Example 32 | Liquid formulation | Saline | HER2/neu_A02 (1.25) | c-di-GMP (0.2) | None | None | 10 µL | once | Genetically modified | 560 |
| Example 33 | Liquid formulation | Saline | HER2/neu_A02 (1.25) | c-di-GMP (0.2) | PEP (0.3) | None | 10 µL | once | Genetically modified | 718 |
| Comparative Example 4 | Subcutaneous injection | Saline | HER2/neu_A02 (0.125) | Montanide ISA51VG (50) | | None | 200 µL | once | Genetically modified | 93 |

**Table 7**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 13 | Liquid formulation | Saline | HBVenv (1.25) | None | None | None | 10 µL | once | Genetically modified | 6 |
| Example 34 | Liquid formulation | Saline | HBVenv (1.25) | c-di-GMP (0.2) | None | None | 10 µL | once | Genetically modified | 542 |
| Example 35 | Liquid formulation | Saline | HBVenv (1.25) | c-di-GMP (0.2) | PEP (0.3) | None | 10 µL | once | Genetically modified | 787 |

**Table 8**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 5 | Liquid formulation | Saline | HER2/neu E75(1.25) | None | None | None | 20 µL | twice | Genetically modified | 3 |
| Example 36 | Liquid formulation | Saline | HER2/neu E75(1.25) | LPS (0.1) | None | None | 20 µL | twice | Genetically modified | 15 |
| Example 37 | Liquid formulation | Saline | HER2/neu E75(1.25) | None | PEP(0.3) | None | 20 µL | twice | Genetically modified | 11 |
| Example 38 | Liquid formulation | Saline | HER2/neu E75 (1.25) | LPS (0.1) | PEP(0.3) | None | 20 µL | twice | Genetically modified | 30 |
| Example 39 | Liquid formulation | Saline | HER2/neu E75 (1.25) | LPS (0.1) | PEP(0.3) | MA (0.05) | 20 µL | twice | Genetically modified | 41 |
| Example 40 | Liquid formulation | Saline | HER2/neu E75 (1.25) | LPS (0.1) | PEP (0.3) | Isostearic acid (0.05) | 20 µL | twice | Genetically modified | 38 |
| Example 41 | Liquid formulation | Saline | HER2/neu E75(1.25) | LPS (0.1) | PEP(0.3) | Lactic acid (0.05) | 20 µL | twice | Genetically modified | 37 |
| Example 42 | Liquid formulation | Saline | HER2/neu E75(1.25) | LPS (0.1) | PEP (0.3) | Citric acid (0.05) | 20 µL | twice | Genetically modified | 38 |
| Example 43 | Liquid formulation | Saline | HER2/neu E75(1.25) | syn-MPL (0.1) | PEP(0.3) | None | 20 µL | twice | Genetically modified | 27 |
| Example 44 | Liquid formulation | Saline | HER2/neu E75(1.25) | Imiquimod (0.3) | PEP(0.3) | None | 20 µL | twice | Genetically modified | 89 |
| Example 45 | Liquid formulation | Saline | HER2/neu E75(1.25) | Resiquimod (0.1) | PEP(0.3) | None | 20 µL | twice | Genetically modified | 177 |
| Example 46 | Liquid formulation | Saline | HER2/neu E75(1.25) | c-di-GMP (0.2) | None | None | 10 µL | once | Genetically modified | 523 |
| Example 47 | Liquid formulation | Saline | HER2/neu E75 (1.25) | c-di-GMP (0.2) | PEP(0.3) | None | 10 µL | once | Genetically modified | 611 |
| Example 48 | Liquid formulation | Saline | HER2/neu E75(1.25) | Levamisole HCl (0.5) | None | None | 20 µL | twice | Genetically modified | 45 |
| Example 49 | Liquid formulation | Saline | HER2/neu E75 (1.25) | Levamisole HCl (0.5) | PEP(0.3) | None | 20 µL | twice | Genetically modified | 70 |
| Example 50 | Liquid formulation | Saline | HER2/neu E75(1.25) | LPS (0.1) | PADRE (0.3) | None | 20 µL | twice | Genetically modified | 33 |
| Example 51 | Film formulation | HPC/PEG/ mannitol | HER2/neu E75 (5) | LPS (0.1) | PEP(0.3) | None | 10 mg | twice | Genetically modified | 32 |
| Example 52 | Orally disintegrating tablet | Gelatin/ mannitol | HER2/neu E75 (5) | LPS (0.1) | PEP (0.3) | None | 10 mg | twice | Genetically modified | 33 |
| Comparative Example 6 | Subcutaneous injection | Saline | HER2/neu E75 (0.125) | Montanide ISA51VG (50) | | None | 200 µL | once | Genetically modified | 110 |

| | | | | | | | | | | |
|---|---|---|---|---|---|---|---|---|---|---|
| Resiquimod: TLR7 and/or TLR8 ligand | | | | | | | | | | |

**Table 9**

| | Dosage form | Composition | | | | | Dose | Administration | Mouse | Result of immunization (ELISPOT average number of spots) |
|---|---|---|---|---|---|---|---|---|---|---|
| | | Base | Antigen peptide | Cellular immunity induction promoter | | Acid | | | | |
| Comparative Example 14 | Liquid formulation | Saline | OVA protein (1.25) | None | None | None | 20 µL | twice | BALB/c | 9 |
| Example 53 | Liquid formulation | Saline | OVA protein (1.25) | LPS (0.1) | None | None | 20 µL | twice | BALB/c | 120 |
| Example 54 | Liquid formulation | Saline | OVA protein (1.25) | LPS (0.1) | PEPB (0.3) | None | 20 µL | twice | BALB/c | 142 |
| Example 55 | Liquid formulation | Saline | OVA protein (1.25) | LPS (0.1) | PEPB (0.3) | MA (0.05) | 20 µL | twice | BALB/c | 405 |
| Example 56 | Liquid formulation | Saline | OVA protein (1.25) | LPS (0.1) | PEPB (0.3) | Isostearic acid (0.05) | 20 µL | twice | BALB/c | 380 |
| Example 57 | Liquid formulation | Saline | OVA protein (1.25) | LPS (0.1) | PEPB (0.3) | Lactic acid (0.05) | 20 µL | twice | BALB/c | 365 |
| Example 58 | Liquid formulation | Saline | OVA protein (1.25) | LPS (0.1) | PEPB (0.3) | Citric acid (0.05) | 20 µL | twice | BALB/c | 345 |
| Example 59 | Liquid formulation | Saline | OVA protein (1.25) | c-di-GMP (0.1) | None | None | 10 µL | once | BALB/c | 457 |
| Example 60 | Liquid formulation | Saline | OVA protein (1.25) | c-di-GMP (0.1) | PEPB (0.3) | None | 10 µL | once | BALB/c | 524 |
| Example 61 | Film formulation | HPC/PEG/ mannitol | OVA protein (5) | LPS (0.1) | PEPB (0.3) | None | 10 mg | twice | BALB/c | 145 |
| Example 62 | Orally disintegrating tablet | Gelatin/ mannitol | OVA protein (5) | LPS (0.1) | PEPB (0.3) | None | 10 mg | twice | BALB/c | 147 |

### Liquid Formulation for Transnasal Administration

Each liquid formulation having each composition shown in Tables 10 to 19 below was produced, and used as an administration sample in a mouse immunity experiment. Specifically, to an antigen peptide and a cellular immunity induction promoter in amounts set forth in Tables 10 to 19 were added 20 parts by weight of an additive (DMSO) and saline as a base material so that the total amount was 100 parts by weight, and the resultant was mixed to prepare a liquid formulation for transnasal administration. The antigen peptide and the cellular immunity induction promoter were obtained from the same companies as in the case of the liquid formulation for sublingual administration described above.

### Mouse Immunity Experiment 2 (Transnasal Administration)

A mouse immunity experiment for the liquid formulation for transnasal administration was performed. The experiment was performed in accordance with the ELISPOT method. Specifically, in a case where the administration was performed once, after mice were anesthetized, liquid formulation was absorbed from nasal cavities, and the mice were fed for 6 days. In a case where the administration was performed twice, the same procedure as above was repeated after 6 days from the first administration. After 6 days from the final administration, the spleen was isolated, and the antigen-specific cellular immunity induction level was evaluated in accordance with the ELISPOT method. The ELISPOT method was performed in the same manner as in mouse immunity experiment 1.

The results of the immunity experiment are shown in Table 10 to 19 below, together with the mice used, the dosage, and the number of administration. The "genetically modified mice" in Tables are genetically modified mice from which the cellular immunity induction owing to HLA-A* 0201 MHC-restricted peptide can be evaluated. For comparison, the results obtained from immunity caused by injection formulations described below (Comparative Examples 2 to 4 and 6 to 8) were described at the end of each Table.

**Table 10**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Comparative Example 15 | Liquid formulation | Saline | Survivin 2B (2.5) | None | None | 10 µL | twice | BALB/c | 3 |
| Example 63 | Liquid formulation | Saline | Survivin 2B (2.5) | LPS (0.1) | None | 10 µL | twice | BALB/c | 15 |
| Example 64 | Liquid formulation | Saline | Survivin 2B (2.5) | LPS (0.1) | PEPB (0.3) | 10 µL | twice | BALB/c | 20 |
| Example 65 | Liquid formulation | Saline | Survivin 2B (2.5) | poly(I:C) (0.1) | PEPB (0.3) | 10 µL | twice | BALB/c | 18 |
| Example 66 | Liquid formulation | Saline | Survivin 2B (2.5) | c-di-GMP (0.2) | PEPB (0.3) | 10 µL | once | BALB/c | 142 |
| Comparative Example 2 | Subcutaneous injection | Saline | Survivin 2B (0.125) | Montanide ISA51VG (50) | | 200 µL | once | BALB/c | 313 |

| | | | | | | | | | |
|---|---|---|---|---|---|---|---|---|---|
| poly (I:C) : Polyinosinic-polycytidylic acid (TLR3 ligand) | | | | | | | | | |

**Table 11**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Comparative Example 16 | Liquid formulation | Saline | MAGE3_A02 (5) | None | None | 10 µL | twice | Genetically modified | 6 |
| Example 67 | Liquid formulation | Saline | MAGE3_A02 (5) | None | PEP (0.3) | 10 µL | twice | Genetically modified | 12 |
| Example 68 | Liquid formulation | Saline | MAGE3_A02 (5) | LPS (0.1) | None | 10 µL | twice | Genetically modified | 42 |
| Example 69 | Liquid formulation | Saline | MAGE3_A02 (5) | LPS (0.1) | PEP(0.3) | 10 µL | twice | Genetically modified | 59 |
| Example 70 | Liquid formulation | Saline | MAGE3_A02 (5) | c-di-GMP (0.2) | PEP(0.3) | 10 µL | once | Genetically modified | 1151 |

**Table 12**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Example 71 | Liquid formulation | Saline | MAGE3_A29 (10) | c-di-GMP (0.2) | PEPB (0.3) | 10 µL | once | BALB/c | 78 |
| Comparative Example 7 | Subcutaneous injection | Saline | MAGE3_A24 (0.125) | Montanide ISA51VG (50) | | 200 µL | once | BALB/c | 56 |

**Table 13**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Example 72 | Liquid formulation | Saline | GPC3 (10) | c-di-GMP (0.2) | PEPB (0.3) | 10 µL | once | BALB/c | 13 |
| Comparative Example 8 | Subcutaneous injection | Saline | GPC3 (0.125) | Montanide ISA51VG (50) | | 200 µL | once | BALB/c | 10 |

**Table 14**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Example 73 | Liquid formulation | Saline | HER2/neu_A24 (10) | c-di-GMP (0.2) | PEPB (0.3) | 10 µL | once | BALB/c | 28 |

**Table 15**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Comparative Example 17 | Liquid formulation | Saline | IPEP87 (10) | None | None | 10 µL | twice | Genetically modified | 6 |
| Example 74 | Liquid formulation | Saline | IPEP87 (10) | None | PEP(0.3) | 10 µL | twice | Genetically modified | 53 |
| Example 75 | Liquid formulation | Saline | IPEP87 (10) | LPS (0.1) | None | 10 µL | twice | Genetically modified | 187 |
| Example 76 | Liquid formulation | Saline | IPEP87 (10) | LPS(0.1) | PEP(0.3) | 10 µL | twice | Genetically modified | 295 |
| Example 77 | Liquid formulation | Saline | IPEP87 (10) | Imiquimod (0.3) | PEP(0.3) | 10 µL | twice | Genetically modified | 229 |
| Example 78 | Liquid formulation | Saline | IPEP87 (10) | c-di-GMP (0.2) | PEP(0.3) | 10 µL | once | Genetically modified | 909 |
| Example 79 | Liquid formulation | Saline | IPEP87 (10) | Levamisole HCl (0.5) | PEP(0.3) | 10 µL | twice | Genetically modified | 235 |

**Table 16**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Comparative Example 18 | Liquid formulation | Saline | PR1 (1.25) | None | None | 10 µL | twice | Genetically modified | 2 |
| Example 80 | Liquid formulation | Saline | PR1 (1.25) | None | PEP(0.3) | 10 µL | twice | Genetically modified | 10 |
| Example 81 | Liquid formulation | Saline | PR1 (1.25) | LPS (0.1) | None | 10 µL | twice | Genetically modified | 15 |
| Example 82 | Liquid formulation | Saline | PR1 (1.25) | LPS (0.1) | PEP(0.3) | 10 µL | twice | Genetically modified | 26 |
| Example 83 | Liquid formulation | Saline | PR1 (1.25) | c-di-GMP (0.2) | PEP(0.3) | 10 µL | once | Genetically modified | 92 |
| Comparative Example 3 | Subcutaneous injection | Saline | PR1 (0.125) | Montanide ISA51VG (50) | | 200 µL | once | Genetically modified | 144 |

**Table 17**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Comparative Example 19 | Liquid formulation | Saline | HER2/neu_A02 (1.25) | None | None | 10 µL | twice | Genetically modified | 5 |
| Example 84 | Liquid formulation | Saline | HER2/neu_A02 (1.25) | None | PEP (0.3) | 10 µL | twice | Genetically modified | 13 |
| Example 85 | Liquid formulation | Saline | HER2/neu_A02 (1.25) | LPS (0.1) | None | 10 µL | twice | Genetically modified | 84 |
| Example 86 | Liquid formulation | Saline | HER2/neu_A02 (1.25) | LPS (0.1) | PEP (0.3) | 10 µL | twice | Genetically modified | 114 |
| Example 87 | Liquid formulation | Saline | HER2/neu_A02 (1.25) | c-di-GMP (0.2) | PEP (0.3) | 10 µL | once | Genetically modified | 678 |
| Comparative Example 4 | Subcutaneous injection | Saline | HER2/neu_A02 (0.125) | Montanide ISA51VG (50) | | 200 µL | once | Genetically modified | 93 |

**Table 18**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Example 88 | Liquid formulation | Saline | HBVenv (1.25) | c-di-GMP (0.2) | PEP(0.3) | 10 µL | once | Genetically modified | 329 |

**Table 19**

| | Dosage form | Composition | | | | Dose | Administration | Mouse | Result of immunization (ELISPOT average number of spots) |
|---|---|---|---|---|---|---|---|---|---|
| | | Base | Antigen peptide | Cellular immunity induction promoter | | | | | |
| Comparative Example 20 | Liquid formulation | Saline | HER2/neu E75 (1.25) | None | None | 10 µL | twice | Genetically modified | 3 |
| Example 89 | Liquid formulation | Saline | HER2/neu E75 (1.25) | None | PEP (0.3) | 10 µL | twice | Genetically modified | 11 |
| Example 90 | Liquid formulation | Saline | HER2/neu E75 (1.25) | LPS (0.1) | None | 10 µL | twice | Genetically modified | 16 |
| Example 91 | Liquid formulation | Saline | HER2/neu E75 (1.25) | LPS (0.1) | PEP(0.3) | 10 µL | twice | Genetically modified | 31 |
| Example 92 | Liquid formulation | Saline | HER2/neu E75 (1.25) | Pam3CSK4 (0.1) | PEP(0.3) | 10 µL | twice | Genetically modified | 69 |
| Example 93 | Liquid formulation | Saline | HER2/neu E75 (1.25) | poly(I:C) (0.1) | PEP(0.3) | 10 µL | twice | Genetically modified | 183 |
| Example 94 | Liquid formulation | Saline | HER2/neu E75 (1.25) | c-di-GMP (0.2) | PEP(0.3) | 10 µL | once | Genetically modified | 476 |
| Example 95 | Liquid formulation | Saline | HER2/neu E75 (1.25) | LPS (0.1) | PADRE (0.3) | 10 µL | twice | Genetically modified | 34 |
| Comparative Example 6 | Subcutaneous injection | Saline | HER2/neu E75 (0.125) | Montanide ISA51VG (50) | | 200 µL | once | Genetically modified | 110 |

| | | | | | | | | | |
|---|---|---|---|---|---|---|---|---|---|
| Pam3CSK4: TLR1/2 ligand | | | | | | | | | |

### Subcutaneous Injection Formulation

Each subcutaneous injection formulation having each composition shown in Table 20 below was produced, and used as an administration sample in an immunity experiment. Specifically, to an antigen peptide and an adjuvant, Montanide ISA51VG (Freund Corporation), in amounts set forth in Table 20, were added 0.5 parts by weight of an additive (DMSO) and saline as a base material so that the total amount was 100 parts by weight, and the resultant was mixed to prepare an injection formulation. The antigen peptide was obtained from the same company as in the case of the liquid formulation for sublingual administration described above.

### Mouse Immunity Experiment 3 (Subcutaneous Injection)

A mouse immunity experiment for the subcutaneous injection formulation described above was performed. The experiment was performed in accordance with the ELISPOT method. Specifically, after 200 µL of the formulation was subcutaneously injection-administered to the back of a mouse, the mouse was raised for 6 days. After 6 days from the day on which the administration was performed, the spleen was extirpated, and the antigen-specific cellular immunity induction level was evaluated in accordance with the ELISPOT method. The number of administrations was once in every case. The ELISPOT method was performed in the same manner as in mouse immunity experiment 1.

The results of the immunity experiment are shown in Table 20 below, together with the mice used. The "genetically modified mice" in Table are genetically modified mice from which the cellular immunity induction owing to HLA-A* 0201 MHC-restricted peptide can be evaluated.

**Table 20**

| | Composition | | | Mouse | Result of immunization (ELISPOT average number of spots) |
|---|---|---|---|---|---|
| | Base | Antigen peptide | Cellular immunity induction promoter | | |
| Comparative Example 2 | Saline | Survivin 2B (0.125) | Montanide ISA51VG(50) | BALB/c | 313 |
| Comparative Example 3 | Saline | PR1 (0.125) | Montanide ISA51VG(50) | Genetically modified | 144 |
| Comparative Example 4 | Saline | HER2/neu_A02 (0.125) | Montanide ISA51VG (50) | Genetically modified | 93 |
| Comparative Example 6 | Saline | HER2/neu E75 (0.125) | Montanide ISA51VG(50) | Genetically modified | 110 |
| Comparative Example 7 | Saline | MAGE3_A24 (0.125) | Montanide ISA51VG(50) | BALB/c | 56 |
| Comparative Example 8 | Saline | GPC3 (0.125) | Montanide ISA51VG (50) | BALB/c | 10 |

In Tables 1-9, a vaccine composition for mucosal administration comprising an antigen and a cellular immunity induction promoter was administered in sublingual route, and an efficacy of the cellular immunity induction promoter was evaluated.

As a result, one or more cellular immunity induction promoters selected from the group consisting of TLR ligand, a cyclic dinucleotide, a helper peptide and an immunomodulatory small molecule drug were effective.

Preferably, a cellular immunity induction promoter selected from TLR4 ligand, TLR7 and/or TLR8 ligand, a cyclic dinucleotide, a helper peptide, an immunomodulatory small molecule drug and a combination of two or more of them was effective.

More preferably, TLR4 ligand, TLR7 and/or TLR8 ligand, a cyclic dinucleotide, and a combination of these compounds with a helper peptide were particularly effective.

From the viewpoint of safety, a sublingual administration is preferable rather than a nasal administration. However, the strong induction of immunity was also confirmed when a film formulation or an orally-disintegrating tablet, which is a preferable form in view of convenient administration and storage stability, was used.

In Tables 10-19, a vaccine composition for mucosal administration comprising an antigen and a cellular immunity induction promoter was administered in nasal route, and an efficacy of the cellular immunity induction promoter was evaluated.

One or more cellular immunity induction promoters selected from the group consisting of TLR ligand, a cyclic dinucleotide, a helper peptide and an immunomodulatory small molecule drug were effective.

Preferably, a cellular immunity induction promoter selected from TLR1/2 ligand, TLR3 ligand, TLR4 ligand, TLR7 and/or TLR8 ligand, a cyclic dinucleotide, a helper peptide, an immunomodulatory small molecule drug and a combination of two or more of them was effective.

## Claims

1. A vaccine composition for mucosal administration for use in the induction of cellular immunity, comprising:
(i) an antigen; and
(ii) at least one more cellular immunity induction promoter selected from the group consisting of a TLR ligand, a cyclic dinucleotide, a helper peptide and an immunomodulatory small molecule drug.

2. The vaccine composition according to claim 1, which is for use in the treatment of a cancer.

3. The vaccine composition according to claim 1, which is for use in the treatment of a viral disease.

4. The vaccine composition according to any one of claims 1 to 3, wherein the cellular immunity induction promoter is a helper peptide.

5. The vaccine composition according to any one of claims 1 to 3, wherein the cellular immunity induction promoter is a combination of a helper peptide and at least one substance selected from the group consisting of a TLR ligand, a cyclic dinucleotide and an immunomodulatory small molecule drug.

6. The vaccine composition according to any one of claims 1 to 5, wherein the antigen is a peptide selected from the group consisting of survivin 2B peptide and/or modified survivin-2B peptide, GPC3 peptide and/or modified GPC3 peptide, HER2/neu_A24 peptide and/or modified HER2/neu_A24 peptide,
MAGE3_A24 peptide and/or modified MAGE3_A24 peptide, IPEP87 peptide and/or modified IPEP87 peptide, PR1 peptide and/or modified PR1 peptide, HER2/neu_A02 peptide and/or modified HER2/neu_A02 peptide, MAGE3_A02 peptide and/or modified MAGE3_A02 peptide, HBVenv peptide and/or modified HBVenv peptide, and MUC1 peptide and/or modified MUC1 peptide.
